# EUROPEAN PATENT SPECIFICATION

(11) **EP 2 918 674 B1**
(45) Date of publication and mention of the grant of the patent: **29.08.2018**
(21) Application number: 15158837.3
(22) Date of filing: 12.03.2015
(51) Int. Cl.: C12M 1/00, C12M 1/12, C12N 5/074, C12M 3/06, C12N 5/00

(54) **Cell culture method, cell culture member, and cell culture apparatus**
Zellkulturverfahren, Zellkulturelement und Zellkulturvorrichtung
Procédé de culture cellulaire, élément de culture cellulaire et appareil de culture cellulaire

(30) Priority: 13.03.2014 JP 2014050629; 16.02.2015 JP 2015027534
(43) Date of publication of application: 16.09.2015
(73) Proprietor: ARKRAY, Inc., Minami-ku Kyoto-shi Kyoto 601-8045 (JP)
(72) Inventor: Matsumura, Taku, Kyoto-shi, Kyoto 602-0008 (JP); Okonogi, Atsuhito, Kyoto-shi, Kyoto 602-0008 (JP); Nakanishi, Naoyuki, Kyoto-shi, Kyoto 602-0008 (JP); Noda, Yuichiro, Kyoto-shi, Kyoto 602-0008 (JP)
(74) Representative: Dehns

(56) References cited:
- WO-A1-2013/058403
- US-A1- 2012 220 031
- ANNA TOUROVSKAIA ET AL: "Differentiation-on-a-chip: A microfluidic platform for long-term cell culture studies", LAB ON A CHIP, vol. 5, no. 1, 1 January 2005 (2005-01-01) , pages 14-9, XP055194753, ISSN: 1473-0197, DOI: 10.1039/b405719h
- HUANG W H ET AL: "Recent advances in single-cell analysis using capillary electrophoresis and microfluidic devices", JOURNAL OF CHROMATOGRAPHY B: BIOMEDICAL SCIENCES & APPLICATIONS, ELSEVIER, AMSTERDAM, NL, vol. 866, no. 1-2, 15 April 2008 (2008-04-15), pages 104-122, XP022595822, ISSN: 1570-0232, DOI: 10.1016/J.JCHROMB.2008.01.030 [retrieved on 2008-02-02]

## Description

### BACKGROUND OF THE INVENTION

### Technical Field

The present invention relates to a cell culture method. Also disclosed is a cell culture member, and a cell culture apparatus.

### Description of the Related Art

In order to obtain a population of cells which are derived from a single cell and which have identical genetic information (hereinafter also referred to as "clone cells"), it is necessary to culture the single cell in a state where the single cell is singly seeded such that contacting of the single cell with another cell or the like is inhibited. A method of obtaining such clone cells is expected to be particularly necessary in cases in which pluripotent cells are used industrially.

Examples of cell culture methods for obtaining clone cells include the method disclosed in WO 13/058403, in which singly dispersed cells are cultured in a microchannel in which laminar flow conditions for the culture fluid are controlled.

WO 11/043405 discloses an invention in which singly dispersed human pluripotent stem cells are cultured in a single-cell state while the pluripotency is maintained, wherein the extracellular-matrix coating of the culture matrix applied is the E8 fragment of human laminin α5β1γ1 or the E8 fragment of human laminin α3β3γ2.

WO 09/123349 discloses a culture method in which culturing is carried out in a medium which contains laminin 5 but does not contain feeder cells or serum, in order to avoid the potential risk of viral contamination and the like while maintaining the pluripotency of pluripotent stem cells.

### SUMMARY OF THE INVENTION

As described above, WO 13/058403 and WO 11/043405 describe that clone cells can be obtained by culturing.

The technique of WO 13/058403 is based on the finding that cloning by singly dispersed cell culturing can be achieved by allowing a culture medium to flow at laminar flow conditions.

In the technique of WO 11/043405, from 1×10³ to 5×10⁴ singly dispersed cells are seeded in a well having a small base area. Therefore, growth might be possible only in cases in which adherence of cells to each other occurs after seeding to form a cluster of two or more cells.

Another possible method of culturing single cells may be a method using an agent called an apoptosis inhibitor (Rock inhibitor), which inhibits apoptosis by cleaving intercellular adhesion. However, addition of an agent or the like is not preferred since the addition might adversely affect the cell quality.

An object of an aspect of the invention is to provide a cell culture method that enables culturing of a single cell in a single-cell state to obtain clone cells thereof.

The present disclosure includes the following embodiments.
<1> A cell culture method comprising:
   a seeding process of singly seeding single pluripotent cells on a coating layer of a microchannel, the coating layer being present on an inner wall of the microchannel, and the coating layer containing laminin; and
   a circulation process of circulating a culture fluid in the microchannel where the single pluripotent cells have been singly seeded in the seeding process, wherein the pluripotency is maintained during the method. Thus, also provided is a cell culture method for maintaining the pluripotency of cells, comprising a seeding process and a circulation process as described herein and the cells are pluripotent cells.
<2> The cell culture method according to <1>, wherein the single pluripotent cells seeded in the seeding process are cells that have been subcultured in a medium containing laminin.
<3> The cell culture method according to <2>, wherein the number of times of the subculturing (number of subculturing passages) is not less than 2.
<4> The cell culture method according to any one of <1> to <3>, wherein the culture fluid is a conditioned culture fluid.
<5> The cell culture method according to any one of <1> to <4>, wherein, in the circulation process, the circulation of the culture fluid is started after maintaining the microchannel, in which the single pluripotent cells have been singly seeded in the seeding process, in a state where the inside of the microchannel is filled with the culture fluid. Preferably, the maintenance is for a predetermined time period. As referred to herein, reference to the microchannel being maintained in a state in which the microchannel is filled with culture fluid means that the flow of the fluid in the microchannel is stopped, i.e. the flow rate is 0. This maintenance may occur for a predetermined time period, for example, 12 hours to 3 days, e.g. around 24 hours.
<6> The cell culture method according to any one of <1> to <5>, wherein the laminin is human laminin.
<7> The cell culture method according to any one of <1> to <6>, wherein the microchannel has, in the inside thereof, an isolating structure which isolates each single pluripotent cell from other cells.

Also disclosed herein, but not forming an object of the invention is a cell culture member comprising a microchannel, the microchannel having a coating layer containing a laminin on an inner wall where single cells are seeded. Preferred examples of laminins are as described hereinafter.

Also disclosed is a cell culture member as described hereinbefore, wherein the microchannel has, in the inside thereof, an isolating structure which isolates each single cell from other cells.

Further disclosed in a cell culture apparatus comprising: the cell culture member described hereinbefore; and a circulation device for circulating a culture fluid in the microchannel of the cell culture member. Preferably the cell culture member or cell culture apparatus is adapted for (or capable of) performance of the cell culture method of the invention. Also disclosed herein is a cell culture method as described herein using a cell culture member or apparatus as described herein as well as use of a cell culture member or apparatus as described herein in a cell culture method described herein or use of a cell culture member or apparatus as described herein for maintaining the pluripotency of cell, particularly in a cell culture method described herein.

According to the present disclosure, it is possible to provide a cell culture method, a cell culture member, and a cell culture apparatus that enable culturing of single cells in a single-cell state to obtain clone cells thereof.

### BRIEF DESCRIPTION OF DRAWINGS

Fig. 1 is a plan view illustrating an example of the whole constitution of the cell culture apparatus as disclosed herein.
Fig. 2 is an exploded perspective view illustrating an example of the cell culture member as disclosed herein.
Fig. 3 is a cross-sectional view illustrating an example of the cell culture member as disclosed herein.
Fig. 4 is a cross-sectional view illustrating another example of the cell culture member as disclosed herein.
Fig. 5 is a cross-sectional view illustrating another example of the cell culture member as disclosed herein.
Fig. 6 is a diagram showing the result of observation of cells cultured by an example of the cell culture method as disclosed herein, which observation was carried out using a phase-contrast microscope.
Fig. 7A is a diagram showing the result of observation of TIG1-iPSCs on the 14th day of culturing (Day 14 of culturing) in a microchannel.
Fig. 7B is a diagram showing the result of observation of TIG1-iPSCs prepared by removing the TIG1-iPSCs in Fig. 7A and subjecting the removed cells to acclimation culture on a Matrigel-coated plate.
Fig. 7C is a diagram showing the result of observation of TIG1-iPSCs prepared by removing the TIG1-iPSCs in Fig. 7B and subjecting the removed cells to the first subculture.
Fig. 8 is a diagram showing the result of immunostaining carried out for confirming maintenance of pluripotency by cells cultured by an example of the cell culture method of the present aspect.

### DETAILED DESCRIPTION OF THE INVENTION

### [Cell Culture Method]

A cell culture method of the present aspect includes: a seeding process of singly seeding single pluripotent cells on a coating layer of a microchannel, the coating layer being present on an inner wall of the microchannel where the single cells are to be seeded, and the coating layer containing laminin; and a circulation process of circulating a culture fluid in the microchannel where the single pluripotent cells have been singly seeded in the seeding process, wherein the pluripotency is maintained during the method.

According to the cell culture method of the present aspect, the seeding process and the circulation process allow a single pluripotent cell to be cultured in a single-cell state, thereby producing clone cells of the single pluripotent cell.

The processes are described below.

### (Seeding Process)

In the seeding process, single cells are singly seeded on a coating layer of a microchannel, which coating layer is present on an inner wall of the microchannel and contains laminin.

The term "single cells" herein means cells each of which is not contacting other cells, and, specifically means isolated cells which are not joined with each other via an extracellular matrix such as cadherin.

The term "seeding single cells in a single-cell state" or "singly seeding single cells" means that single cells are seeded in a state where each cell is not in contact with another cell and where contacting of each cell with another cell hardly occurs.

Examples of the method of seeding single cells in a single-cell state include a method in which a single-cell dispersion liquid, in which single cells are dispersed, is injected into a microchannel having a coating layer (hereinafter also referred to as "microchannel").

Specific examples of the method include: (1) a method in which a single-cell dispersion liquid having a low single-cell concentration is injected into a microchannel; and/or (2) a method in which a single-cell dispersion liquid having a low single-cell concentration is injected into a microchannel having an isolating structure which isolates each single cell from other cells.

Details of the isolating structure is described in the section on the cell culture member. In general terms the isolating structure is a structure which serves to contain the single cell and provides a physical constraint or barrier to contain and isolate that cell and thereby separates each single cell from other cells.

The distance between the single cells seeded in a single-cell state are preferably not less than 1 mm, more preferably from 1 mm to 10 mm, to allow seeding in a single-cell state.

The single-cell dispersion liquid contains single cells, a solvent, and one or more other additives.

Examples of the solvent include a culture fluid. The culture fluid is preferably the same as the culture fluid to be circulated in the circulation process described below.

The cell concentration in the single-cell dispersion liquid is preferably low to allow seeding of single cells in a single-cell state.

More specifically, the cell concentration in the single-cell dispersion is, for example, preferably from 2.5×10² cells/ml to 1.0×10⁴ cells/ml, more preferably from 5.0×10² cells/ml to 5.0×10³ cells/ml, particularly preferably 5.0×10² cells/ml.

The distance between the single cells seeded can be controlled by the cell concentration in the single-cell dispersion liquid.

Examples of the method of preparing the single-cell dispersion liquid, that is, the method of obtaining the single cells, include known methods of isolating a single cell from a population of 2 or more cells joined with each other via cadherin or the like. Examples of such known methods include mechanical methods and methods using an agent.

Examples of the agent include reagents containing an enzyme having protease activity or EDTA (ethylenediaminetetraacetic acid). Specific examples of the reagents include, but are not limited to, trypsin/EDTA, ACCUTASE (registered trademark, manufactured by Bioneer), and ACCUMAX (registered trademark, manufactured by Bioneer).

The cells cultured by the cell culture method of the present aspect are described below.

The coating layer on which the single cells are singly seeded contains laminin.

Laminin is a known glycoprotein which is a major component of the basal membrane. Any protein identified as laminin may be used. Laminins are heterotrimeric proteins that contain an α-chain, a β-chain, and a γ-chain, found in five, four, and three genetic variants, respectively. The laminin molecules are named according to their chain composition. Laminin-511, for example contains α5, β1, and γ1 chains, whereas laminin-521 contains α5, β2, and γ1 chains. The laminin does not need to be a purified product, and, for example, a basal membrane extract containing laminin as a major component may be used.

The laminin may be derived from any of human, mouse, and rat. Human-derived laminin is particularly preferred.

Examples of the human-derived laminin include human laminin 521, human laminin 511, human laminin 522, and human laminin 523. The human-derived laminin is preferably human laminin 521 or human laminin 511, particularly preferably human laminin 521.

Details of the coating layer are described below.

The single cells to be seeded in a single-cell state are preferably cells obtained by subculturing in a medium containing laminin.

That is, the single cells to be seeded in a single-cell state are preferably single cells separated by subjecting a population of cells cultured in a medium containing laminin to the method of preparing a single-cell dispersion liquid described above.

Examples of the laminin include the same type of laminin as the laminin contained in the coating layer on which the single cells are singly seeded.

The laminin contained in the subculture medium is preferably the same type of laminin as the laminin for the coating layer of the microchannel.

Examples of the medium containing laminin to be used for the subculturing include media on a solid phase having a laminin molecule at least on the surface thereof, more specifically, plates coated with laminin. Plates coated with laminin are commercially available as animal cell culture plates, and these commercially available laminin-coated plates may be preferably used in the method of the present aspect.

The number of times of subculturing (culture passages) in the laminin-containing medium is preferably as large as possible, and particularly preferably not less than 2.

That is, the single cells to be seeded in a single-cell state are preferably single cells separated by subjecting a cell population prepared by performing at least 2 times of successive subculturing in a medium containing laminin to the method of preparing a single-cell suspension described above.

The cells cultured in the cell culture method of the present aspect is pluripotent cells. In methods not according to the invention, the methods disclosed herein may be applied to other types of cells.

The term "pluripotent cells" used in the present disclosure means cells having ability to differentiate into a plurality of types of cells. Examples of the pluripotent cells include, but are not limited to, (A) embryonic stem cells (ES cells), (B) germline stem cells (GS cells), (C) embryonic germ cells (EG cells), (D) induced pluripotent stem cells (iPS cells), (E) ES cells derived from a non-human clone embryo obtained by nuclear transplantation, and (F) pluripotent cells derived from cultured fibroblasts or bone marrow stem cells (Multilineage-differentiating Stress Enduring cells, Muse cells). The source of the pluripotent cells may be selected from various organisms. The pluripotent cells are preferably derived from a mammal such as a human, more preferably derived from mouse or primate. The pluripotent cells are most preferably derived from a human. Described herein are human embryonic cells which may be obtained by destruction of embryos. The invention does not extend to any methods or products which involve the destruction of human embryos and in particular does not extend to the use of human ES cells obtained by destruction of embryos. In a preferred feature when ES cells are used they are preferably non-human.

The cells (A) to (F) are described below for reference.

### (A) Embryonic Stem Cells

ES cells are stem cells that can be established from the inner cell mass of an early embryo (for example, blastocyst) of a mammal such as human or mouse, which cells have pluripotency and growth ability by self-renewal. ES cells can be embryo-derived stem cells originated from the inner cell mass of a blastocyst, which is the embryo formed following the 8-cell stage and the morula stage of a fertilized egg, and ES cells have ability to differentiate into any cells of the cells constituting an adult, that is, the so called pluripotency of differentiation, and growth ability by self-renewal. ES cells were discovered in mouse in 1981 (M. J. Evans and M. H. Kaufman (1981), Nature 292:154-156), and this was followed by establishment of ES cell lines of primates such as human and monkey (J. A. Thomson et al. (1998), Science 282:1145-1147; J. A. Thomson et al. (1995), Proc. Natl. Acad. Sci. USA, 92:7844-7848; J. A. Thomson et al. (1996), Biol. Reprod., 55:254-259; J. A. Thomson and V. S. Marshall (1998), Curr. Top. Dev. Biol., 38:133-165).

ES cells can be established by removing the inner cell mass from the blastocyst of a fertilized egg of a subject animal, followed by culturing the inner cell mass on feeder fibroblasts. The cells can be maintained by subculturing using a culture fluid supplemented with a substance(s) such as leukemia inhibitory factor (LIF) and/or basic fibroblast growth factor (bFGF). Methods of establishment and maintenance of human and monkey ES cells are described in, for example, US 5,843,780 B; Thomson JA, et al. (1995), Proc Natl. Acad. Sci. USA. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; and Klimanskaya I, et al. (2006), Nature. 444:481-485.

With respect to the culture fluid for preparation of ES cells, human ES cells can be maintained, for example, using DMEM (Dulbecco's Modified Eagle's Medium)/F-12 culture fluid supplemented with 0.1 mM 2-mercaptoethanol, 0.1 mM non-essential amino acids, 2 mM L-glutamic acid, 20% KSR (Kinase suppressor of ras), and 4 ng/ml bFGF at 37°C under a moist atmosphere of 2% CO₂/98% air (O. Fumitaka et al. (2008), Nat. Biotechnol., 26:215-224). The ES cells need to be subcultured every 3 to 4 days, and the subculturing can be carried out using, for example, 0.25% trypsin and 0.1 mg/ml collagenase IV in PBS (phosphate buffered saline) containing 1 mM CaCl₂ and 20% KSR.

Selection of the ES cells can be generally carried out by Real-Time PCR using as an index/indices expression of a gene marker(s) such as alkaline phosphatase, Oct-3/4, and/or Nanog. In particular, for selection of human ES cells, expression of a gene marker(s) such as OCT-3/4, NANOG, and/or ECAD can be used as an index/indices (E. Kroon et al. (2008), Nat. Biotechnol., 26:443-452).

With respect to human ES cell lines, for example, reference cell lines WA01(H1) and WA09(H9) can be obtained from WiCell Research Institute, and reference cell lines KhES-1, KhES-2, and KhES-3 can be obtained from Institute for Frontier Medical Sciences, Kyoto University (Kyoto, Japan).

### (B) Germline Stem Cells

Germline stem cells are pluripotent stem cells derived from testis, and play a role as the origin for spermatogenesis. Similarly to ES cells, germline stem cells can be induced to differentiate into various series of cells, and, for example, have the ability to enable preparation of a chimeric mouse by transplantation of the cells to a mouse blastocyst (M. Kanatsu-Shinohara et al. (2003) Biol. Reprod., 69:612-616; K. Shinohara et al. (2004), Cell, 119:1001-1012). Germline stem cells are capable of self-renewal in a culture fluid containing glial cell line-derived neurotrophic factor (GDNF), and, by repeating subculture under the same culture conditions as the conditions for ES cells, germline stem cells can be obtained (Masanori Takehashi et al. (2008), Experimental Medicine, 26(5) (extra edition):41-46, Yodosha (Tokyo, Japan)).

### (C) Embryonic Germ Cells

Embryonic germ cells are established from fetal primordial germ cells and have pluripotency similarly to ES cells. Embryonic germ cells can be established by culturing primordial germ cells in the presence of substances such as LIF, bFGF, and stem cell factor (Y. Matsui et al. (1992), Cell, 70:841-847; J. L. Resnick et al. (1992), Nature, 359:550-551).

### (D) Induced Pluripotent Stem Cells

Induced pluripotent stem (iPS) cells can be prepared by introducing specific reprogramming factors to somatic cells, which reprogramming factors are in the form of DNA or protein. iPS cells are somatic cell-derived artificial stem cells having properties almost equivalent to the properties of ES cells, such as pluripotency of differentiation and growth ability by self-renewal (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al., Nat. Biotechnol. 26:101-106 (2008); WO 2007/069666). The reprogramming factors may be constituted with constituents selected from the group consisting of genes, gene products thereof, and non-coding RNAs specifically expressed in ES cells; and genes, gene products thereof, non-coding RNAs, and low molecular weight compounds which play important roles in maintenance of the undifferentiated state of ES cells. Examples of the genes which may be contained in the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3 and Glis1, and these reprogramming factors may be used singly, or in combination of two or more kinds thereof. Examples of the combination of the reprogramming factors include the combinations described in WO 2007/069666; WO 2008/118820; WO 2009/007852; WO 2009/032194; WO 2009/058413; WO 2009/057831; WO 2009/075119; WO 2009/079007; WO 2009/091659; WO 2009/101084; WO 2009/101407; WO 2009/102983; WO 2009/114949; WO 2009/117439; WO 2009/126250; WO 2009/126251; WO 2009/126655; WO 2009/157593; WO 2010/009015; WO 2010/033906; WO 2010/033920; WO 2010/042800; WO 2010/050626; WO 2010/056831; WO 2010/068955; WO 2010/098419; WO 2010/102267; WO 2010/111409; WO 2010/111422; WO 2010/115050; WO 2010/124290; WO 2010/147395; WO 2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26:2467-2474; Huangfu D, et al. (2008), Nat Biotechnol. 26:1269-1275; Shi Y, et al. (2008), Cell Stem Cell, 3, 568-574; Zhao Y, et al. (2008), Cell Stem Cell, 3:475-479; Marson A, (2008), Cell Stem Cell, 3, 132-135; Feng B, et al. (2009), Nat Cell Biol. 11:197-203; R.L. Judson et al., (2009), Nat. Biotech., 27:459-461; Lyssiotis CA, et al. (2009), Proc Natl Acad Sci USA. 106:8912-8917; Kim JB, et al. (2009), Nature. 461:649-643; Ichida JK, et al. (2009), Cell Stem Cell. 5:491-503; Heng JC, et al. (2010), Cell Stem Cell. 6:167-74; Han J, et al. (2010), Nature. 463:1096-100; Mali P, et al. (2010), Stem Cells. 28:713-720; and Maekawa M, et al. (2011), Nature. 474:225-9.

Examples of the above-described reprogramming factors also include histone deacetylase (HDAC) inhibitors [for example, low molecular weight inhibitors such as valproic acid (VPA), trichostatin A, sodium butyrate, MC 1293, and M344; and nucleic acid-type expression inhibitors such as siRNAs and shRNAs against HDAC (for example, HDAC1 siRNA Smartpool (Millipore) and HuSH 29mer shRNA Constructs against HDAC1 (OriGene))], MEK inhibitors (for example, PD184352, PD98059, U0126, SL327, and PD0325901), Glycogen synthase kinase-3 inhibitors (for example, Bio and CHIR99021), DNA methyltransferase inhibitors (for example, 5'-azacytidine), histone methyltransferase inhibitors (for example, low molecular weight inhibitors such as BIX-01294, and nucleic acid-type expression inhibitors such as siRNAs and shRNAs against Suv39hl, Suv39h2, SetDB1, and G9a), L-channel calcium agonists (for example, Bayk8644), butyric acid, TGFβ inhibitors or ALK5 inhibitors (for example, LY364947, SB431542, 616453, and A-83-01), p53 inhibitors (for example, siRNAs and shRNAs against p53), ARID3A inhibitors (for example, siRNAs and shRNAs against ARID3A), miRNAs such as miR-291-3p, miR-294, miR-295, and mir-302, Wnt Signaling (for example, soluble Wnt3a), neuropeptide Y, prostaglandins (for example, prostaglandin E2 and prostaglandin J2), hTERT, SV40LT, UTF1, IRX6, GLIS1, PITX2, and DMRTB1, which are employed for enhancing the establishment (iPS generation) efficiency. In the present description, these factors employed for the purpose of enhancement of the establishment efficiency are not particularly distinguished from the reprogramming factors.

In cases in which the reprogramming factors are in the form of protein, the reprogramming factors may be introduced into somatic cells by a method such as lipofection, fusion with a cell membrane-permeable peptide (for example, HIV-derived TAT or polyarginine), or microinjection. In cases in which the reprogramming factors are in the form of DNA, the reprogramming factors may be introduced into somatic cells by, for example, use of a vector such as a virus, plasmid, or artificial chromosome; lipofection; use of liposome; or microinjection. Examples of the virus vector include retrovirus vectors, lentivirus vectors (these are described in Cell, 126, pp. 663-676, 2006; Cell, 131, pp. 861-872, 2007; and Science, 318, pp. 1917-1920, 2007), adenovirus vectors (Science, 322, 945-949, 2008), adeno-associated virus vectors, and Sendai virus vectors (WO 2010/008054). Examples of the artificial chromosome vector include human artificial chromosomes (HACs), yeast artificial chromosomes (YACs), and bacterial artificial chromosomes (BACs and PACs). Examples of the plasmid which may be used include plasmids for mammalian cells (Science, 322:949-953, 2008). The vector may contain one or more of regulatory sequences selected from the group consisting of promoters, enhancers, ribosome binding sequences, terminators, and polyadenylation sites, to enable expression of the nuclear reprogramming factors, and may also contain, if necessary, one or more of sequences of selection markers selected from the group consisting of drug resistance genes (for example, kanamycin-resistant gene, ampicillin-resistant gene, and puromycin-resistant gene), thymidine kinase gene, and diphtheria toxin gene; and gene sequences of reporters such as the green-fluorescent protein (GFP), β-glucuronidase (GUS), and FLAG. In order to remove, after introduction of the above vector into somatic cells, the genes encoding the reprogramming factors, or both the promoters and the genes encoding the reprogramming factors linked thereto, the vector may have LoxP sequences upstream and downstream of these sequences.

In cases in which the reprogramming factors are in the form of RNA, each reprogramming factor may be introduced into somatic cells by a method such as lipofection or microinjection, and an RNA into which 5-methylcytidine and pseudouridine (TriLink Biotechnologies) are incorporated may be used in order to suppress degradation (Warren L, (2010) Cell Stem Cell. 7:618-630).

Examples of the culture fluid for induction of the iPS cells include the DMEM, DMEM/F12, and DME culture fluids supplemented with from 10 to 15% FBS (these culture fluids may further contain LIF, penicillin/streptomycin, puromycin, L-glutamine, non-essential amino acids, β-mercaptoethanol and/or the like, as appropriate); and commercially available culture fluids [for example, a culture fluid for culturing mouse ES cells (TX-WES culture fluid, Thromb-X), culture fluid for culturing primate ES cells (culture fluid for primate ES/iPS cells, ReproCELL), and serum-free culture medium (mTeSR, Stemcell Technology)].

Examples of the culture method include a method in which somatic cells and reprogramming factors are brought into contact with each other at 37°C in the presence of 5% CO₂ on DMEM or DMEM/F12 culture fluid supplemented with 10% FBS, and the cells are then cultured for about from 4 to 7 days, followed by seeding the cells on feeder cells (for example, mitomycin C-treated STO cells or SNL cells) and starting culture in a bFGF-containing culture fluid for culturing primate ES cells about 10 days after the contact between the somatic cells and the reprogramming factors, thereby allowing iPS-like colonies to appear from about 30 to about 45 days after the contact, or later.

Alternatively, the cells may be cultured at 37°C in the presence of 5% CO₂ on feeder cells (for example, mitomycin C-treated STO cells or SNL cells) in the DMEM culture fluid supplemented with 10% FBS (this culture fluid may further contain LIF, penicillin/streptomycin, puromycin, L-glutamine, non-essential amino acids, β-mercaptoethanol and/or the like, if necessary) for from about 25 to about 30 days or longer, to allow ES-like colonies to appear. Preferred examples of the culture method include a method in which the somatic cells themselves to be reprogrammed are used instead of the feeder cells (Takahashi K, et al. (2009), PLoS One. 4:e8067, or WO 2010/137746), and a method in which an extracellular matrix (for example, Laminin-5 (WO 2009/123349) or Matrigel (BD)) is used instead.

Other examples of the culture method include a method in which culture is carried out using a serum-free medium (Sun N, et al. (2009), Proc Natl Acad Sci USA. 106:15720-15725). In order to enhance the establishment efficiency, iPS cells may be established under low oxygen conditions (at an oxygen concentration of from 0.1% to 15%) (Yoshida Y, et al. (2009), Cell Stem Cell. 5:237-241 or WO 2010/013845).

During the culture, the culture fluid is replaced with a fresh culture fluid once every day from the second day of the culturing (Day 2 of the culturing). The number of somatic cells used for nuclear reprogramming is not restricted, and usually within the range of from about 5×10³ to about 5×10⁶ cells per 100-cm² area on the culture dish.

iPS cells can be selected based on the shape of each formed colony. In the case in which a drug resistance gene to be expressed in conjunction with a gene that is expressed when a somatic cell is reprogrammed (for example, Oct3/4 or Nanog) is introduced as a marker gene, established iPS cells can be selected by culturing the cells in a culture fluid containing the corresponding drug (selection culture fluid). iPS cells can be selected by observation under a fluorescence microscope in the case in which the marker gene is the gene of a fluorescent protein; by adding a luminescent substrate in the case in which the marker gene is the gene of luciferase; or by adding a colouring substrate in the case in which the marker gene is the gene of a colouring enzyme.

The term "somatic cells" used in the present description means any animal cells (preferably cells of a mammals such as human) excluding germ-line cells and totipotent cells such as eggs, oocytes and ES cells. Examples of the somatic cells include, but are not limited to, any of fetal somatic cells, neonatal somatic cells, and healthy or diseased mature somatic cells, as well as any of primary cultured cells, subcultured cells, and established cell lines. Specific examples of the somatic cells include (1) tissue stem cells (somatic stem cells) such as neural stem cells, hematopoietic stem cells, mesenchymal stem cells and dental pulp stem cells; (2) tissue progenitor cells; and (3) differentiated cells such as lymphocytes, epithelial cells, endothelial cells, muscle cells, fibroblasts (skin cells and the like), hair cells, hepatic cells, gastric mucosal cells, enterocytes, spleen cells, pancreatic cells (pancreatic exocrine cells and the like), brain cells, lung cells, kidney cells, and adipocytes.

In the case in which iPS cells are used as a material for cells to be transplanted, the transplantation is preferably carried out using somatic cells whose HLA genotype is the same or substantially the same as that of the individual to which the cells are to be transplanted, to prevent a rejection reaction. The term "substantially the same" herein means that the HLA genotype is matching to the extent that the immune reaction against the transplanted cells can be suppressed with an immunosuppressive agent. For example, the somatic cells have matched HLA types at the 3 loci HLA-A, HLA-B, and HLA-DR, or at the 4 loci further including HLA-C.

### (E) ES Cells Derived from non-human

Cloned Embryo Obtained by Nuclear Transfer ntES cells are ES cells derived from a non-human cloned embryo prepared by the nuclear transfer technique, and have almost the same properties as the properties of ES cells derived from fertilized eggs (T. Wakayama et al. (2001), Science, 292:740-743; S. Wakayama et al. (2005), Biol. Reprod., 72:932-936; J. Byrne et al. (2007), Nature, 450:497-502). That is, an ntES (nuclear transfer ES) cell is an ES cell established from the inner cell mass of a blastocyst derived from a cloned embryo obtained by replacement of the nucleus of an unfertilized egg with the nucleus of a somatic cell. For preparation of an ntES cell, a combination of the nuclear transfer technique (J. B. Cibelli et al. (1998), Nature Biotechnol., 16:642-646) and the ES cell preparation technique (described above) is employed (Sayaka Wakayama et al. (2008), Experimental Medicine 26(5) (extra edition), pp. 47-52). In the nuclear transfer, reprogramming can be achieved by injecting the nucleus of a somatic cell into a mammalian enucleated unfertilized egg and culturing the resultant for several hours.

### (F) Pluripotent Cells Derived from Cultured Fibroblasts or Bone Marrow Stem Cells (Muse Cells)

Muse cells are pluripotent stem cells produced by the method described in WO 2011/007900. More specifically, Muse cells are cells having pluripotency obtained by subjecting fibroblasts or bone marrow stromal cells to trypsin treatment for a long period, preferably for 8 hours or 16 hours, and then to suspension culture. Muse cells are positive for SSEA-3 and CD105.

### (Circulation Process)

In the circulation process, a culture fluid is allowed to circulate in the microchannel where the single cells have been singly seeded in the seeding process.

The flow rate of the culture fluid to be circulated is preferably from 250 nl/minute to 40,000 nl/minute, more preferably from 500 nl to 10,000 nl/minute, particularly preferably 5000 nl/minute, from the viewpoint of supplying nutrition to the cells while removing waste products.

The type of the culture fluid to be circulated may be selected according to the type of the cells to be cultured. Examples of the culture fluid used for culturing ES cells include DMEM (Dulbecco's Modified Eagle's Medium)/F-12 culture fluid supplemented with 0.1 mM 2-mercaptoethanol, 0.1 mM non-essential amino acids, 2 mM L-glutamic acid, 20% KSR (Kinase suppressor of ras), and 4 ng/ml bFGF (basic fibroblast growth factor). Examples of the culture fluid used for culturing iPS cells include DMEM, DMEM/F12, and DME culture fluid which contain from 10% to 15% FBS (Fetal bovine serum). The examples also include commercially available culture fluid (such as culture fluid for culturing mouse ES cells and culture fluid for culturing primate ES cells) and serum-free media.

A possible example of the method for culturing single cells in a single-cell state is a method in which an agent called apoptosis inhibitor (Rock inhibitor), which inhibits apoptosis by cleaving intercellular adhesion, is added to the culture fluid, but such a method is not preferred since the addition of the agent might adversely affect the cell quality.

In contrast, the cell culture method of the present aspect enables culturing of single cells seeded in a single-cell state, without use of an apoptosis inhibitor.

The culture fluid to be circulated is preferably a conditioned culture fluid. The conditioned culture fluid may be a conditioned culture fluid containing culture supernatant of feeder cells. A preferred example of the conditioned culture fluid is a conditioned culture fluid produced by subculturing of the single cells to be cultured by the method of the present aspect, or a conditioned culture fluid produced by culturing the same type of cells as the single cells.

It is thought that, by the use of the conditioned culture fluid as the culture fluid to be circulated, the single cells seeded in a single-cell state can be easily grown since the phenomenon in which a substance (for example, a substance required for the growth) secreted from a cell acts on a cell other than the cell from which the substance was secreted (paracrine) easily occurs.

In the circulation process, the circulation of the culture fluid is preferably started after maintaining the microchannel, in which the single cells have been singly seeded in the seeding process, in a state where the inside of the microchannel is filled with the culture fluid, for a predetermined time period, for example, from half a day to 3 days. The secretion which is sufficient for the culturing is not secreted from the cells in less than half a day, while the problem of lack of O₂ and nutrients occurs after more than 3 days.

It is thought that this process keeps the culture fluid in the vicinity of the singly seeded single cells, and, as a result, the phenomenon in which a substance (for example, a substance required for the growth) secreted from a cell acts on the cell itself from which the substance was secreted (autocrine) easily occurs, promoting growth of the singly seeded single cells.

The period of maintaining the microchannel in a state where the inside of the microchannel is filled with the culture fluid is, for example, preferably not less than 8 hours, more preferably from 8 hours to 24 hours.

As described above, the cell culture method of the present aspect is preferably carried out at a temperature suitable for culturing the cells. For example, in the case in which the cells are human-derived cells, the temperature of the culture fluid is preferably kept at from 36°C to 38°C so that the cells can be cultured at a temperature close to the body temperature of a human. The cell culture method of the present aspect is preferably carried out under a CO₂ atmosphere of from 4% to 6%.

The cells may be placed under hypoxic conditions for maintenance culture. The hypoxic conditions herein means conditions in which the oxygen partial pressure is from 1% to 10%, preferably 5%.

### [Cell Culture Apparatus]

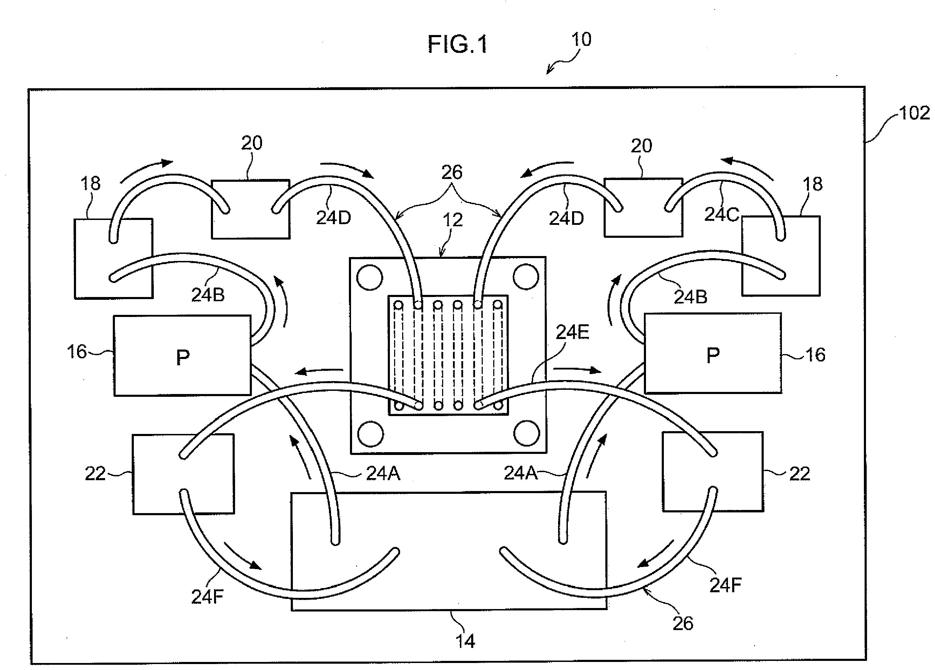

As shown in Fig. 1, the cell culture apparatus 10, which is an example of the cell culture apparatus disclosed herein, includes, for example, the cell culture member 12 mentioned below and a circulation device for circulating a culture fluid in microchannels of the cell culture member 12.

The cell culture method of the present aspect described above can be realized by, for example, using the cell culture apparatus 10 shown in Fig. 1. An apparatus (and the cell culture member therein) as set forth in the Figures and as described below forms a preferred apparatus for performing the method.

The cell culture apparatus disclosed herein is described below in detail with reference to Fig. 1.

### (Circulation Device)

The circulation device comprises, for example, a reservoir (storage section) 14, circulation pumps (liquid transferring means) 16, pressure equalization mechanisms (pressure equalization means) 18, air traps (bubble removing means) 20, and pressurization mechanisms (pressurizing means) 22, and the sections are connected to tubes 24A to 24F to constitute circulation channels 26. The circulation device is provided with two each of circulation pumps 16, pressure equalization mechanisms 18, air traps 20, and pressurization mechanisms 22, so that the culturing can be carried out using 2 channels out of the 6 channels formed in the cell culture member 12. The number of circulation channels 26 is not limited, and a larger number of circulation channels 26 may be provided according to the number of channels used. A plurality of tubes 24 may be connected to one circulation pump 16.

The reservoir 14 is provided in the lower side in Fig. 1, and a culture fluid is stored in the reservoir 14. In this embodiment, 2 circulation pumps 16 are connected to the single reservoir 14. However, the number of circulation pumps connected is not limited, and an independent reservoir 14 may be provided for each circulation pump 16.

To the reservoir 14, circulation pumps 16 as liquid transferring means are connected through the tubes 24A. Although various liquid feed pumps may be used as the circulation pumps 16, small low-flow-rate pumps are preferred. In this embodiment, examples of the circulation pumps 16 include peristaltic tubing pumps, but the pumps are not limited thereto, and other pumps may be used.

The circulation pumps 16 suck the culture fluid from the reservoir 14 through the tubes 24A, and transfer the culture fluid to the tubes 24B. This may enable pushing and forwarding the culture fluid in the pressure equalization mechanisms 18 connected with the tubes 24B to the air traps 20 through the tubes 24C. Similarly, the culture fluid is transferred to the cell culture member 12 through the tubes 24D, and further transferred to the reservoir 14 through the tubes 24E and the pressurization mechanisms 22. Thus, the culture fluid circulates as a laminar flow in the microchannels 32B of the cell culture member 12. The laminar flow herein means that the streamline of the fluid is parallel to the wall surface, and means a flow field which is not a turbulent flow. Preferably, in the flow field, the closer the laminar flow is to the wall, the lower the flow rate is, and, in the area where the laminar flow is more than a certain distance away from the wall, the flow rate is uniform.

The circulation device at least has a tube for connection to a circulation pump (liquid transferring means) 16, and not necessarily a reservoir (storage section) 14, pressure equalization mechanism (pressure equalization means) 18, air trap (bubble removing means) 20, and pressurization mechanism (pressurizing means) 22.

### (Cell Culture Member)

The cell culture member 12 includes microchannels having a coating layer on the inner wall where the single cells are to be seeded.

The cell culture member 12 is concretely described below.

To the cell culture method of the present aspect described above, the cell culture member disclosed herein is preferably applied.

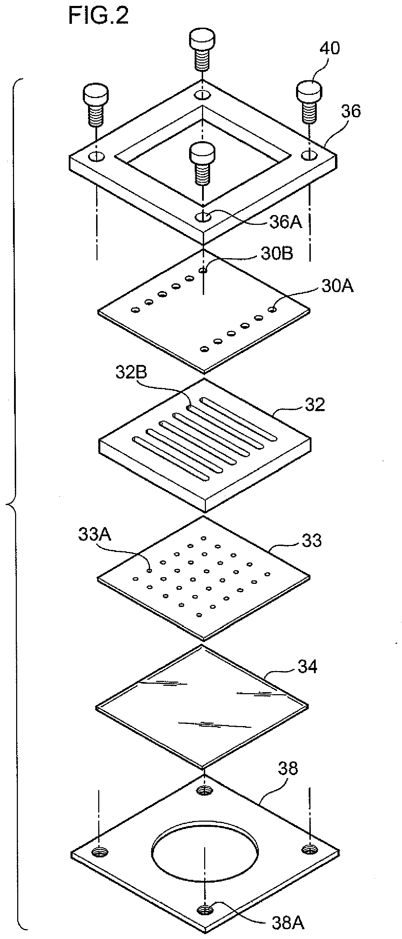

An example of the cell culture member 12 is described below by reference to Fig. 2 to Fig. 5. However, the cell culture member 12 is not limited to the cell culture members shown in Fig. 2 to Fig. 5.

As shown in Fig. 2, the cell culture member 12 includes a resin plate 30, a first polymethylsiloxane (PDMS) plate 32, a second polymethylsiloxane (Poly(dimethylsiloxane) (PDMS)) plate 33, and a glass plate 34, which are stacked in this order. A lower clamp 38 is placed under the glass plate 34, and an upper clamp 36 is placed over the resin plate 30, such that the resin plate 30, the first polymethylsiloxane (PDMS) plate 32, the second polymethylsiloxane (PDMS) plate 33, and the glass plate 34 are sandwiched between the upper clamp 36 and the lower clamp 38. In this state, bolts 40 are inserted through bolt holes 36A formed in the upper clamp 36 and bolt holes 38A formed in the lower clamp 38 to tightly bind the clamps to each other, to thereby form the cell culture member 12. In the lower clamp 38, a hole for observation of cells during culture is formed.

In the resin plate 30, 6 first holes 30A are formed at the positions corresponding, when the resin plate 30 is placed on the first PDMS plate 32, to one end of slits 32B (hereinafter also referred to as "microchannels 32B"), and 6 second holes 30B are formed at the positions corresponding to the other end of the microchannels 32B. By providing the first holes 30A and the second holes 30B at such positions, the culture fluid to be circulated in the microchannels 32B can be made, for example, to pass through tubes 24D to flow into the microchannels 32B from the first holes 30A, followed by flowing through the microchannels 32B and then passing through tubes 24E to flow out from the second holes 30B.

For example, in the first PDMS plate 32, 6 slits 32B for formation of microchannels are independently formed. It should be noted that the number of slits 32B is not limited to 6.

The width of each slit 32B is preferably from 0.1 mm to 1 mm, more preferably from 0.2 mm to 0.5 mm. The depth of each slit 32B is more preferably from 0.1 mm to 1 mm, particularly preferably from 0.2 mm to 0.5 mm. The length of each slit 32B is preferably from 0.5 cm to 10 cm, more preferably from 1 cm to 2 cm.

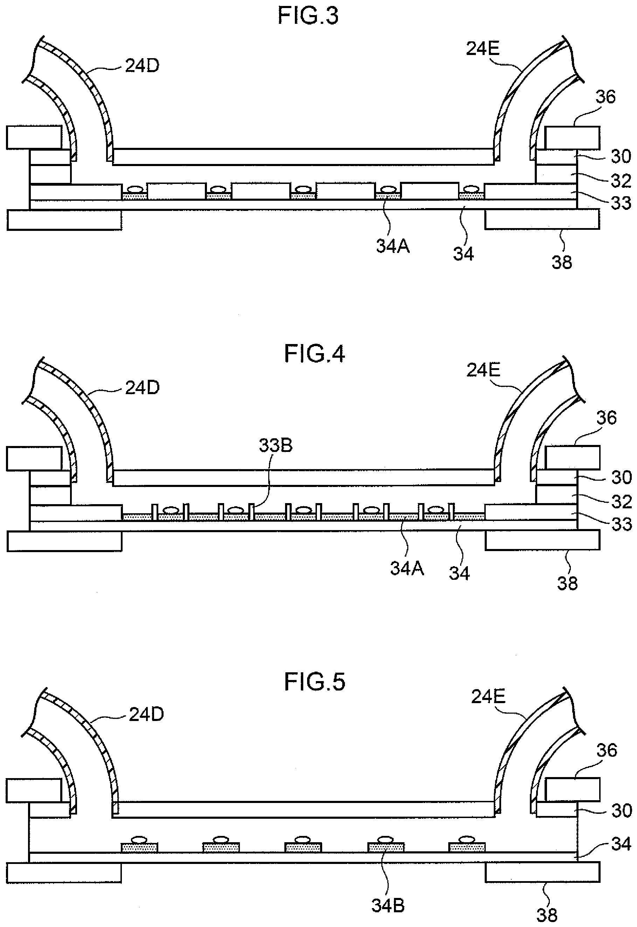

In the second PDMS plate 33, 5 pores (isolating pores 33A) are independently formed for each microchannel 32B of the first PDMS plate 32 such that the pores are positioned along the microchannel when the second PDMS plate 33 is placed on the first PDMS plate 32. Accordingly, as shown in Fig. 3, the isolating pores 33A form pocket-shaped hollows along the microchannel 32B. Thus, the microchannel 32B is a channel having the pocket-shaped hollows.

That is, in the cell culture members 12 shown in Fig. 2 and Fig. 3, pocket-shaped hollows are formed in each microchannel 32B as an isolating structure that isolates each single cell from other cells. The number of isolating pores 33A is not limited to 5.

By seeding a single cell into the inside of each hollow formed by an isolating pore 33A, the cell can be easily cultured in a single-cell state in which the cell is isolated from other cells.

The inner diameter of each isolating pore 33A is preferably from 0.1 mm to 2 mm, more preferably from 0.5 mm to 1.5 mm, particularly preferably from 0.5 mm to 1 mm.

The depth of each isolating pore 33A is preferably from 0.5 mm to 2 mm, and more preferably from 0.5 mm to 1 mm.

The center distance between isolating pores 33A is preferably from 0.1 mm to 20 mm, more preferably from 0.2 mm to 10 mm.

Each of the first PDMS plate 32 and the second PDMS plate 33 may be a plate made of another type of material on which the slits 32B or isolating pores 33A are formed. Examples of the another type of material include plastics, silicone resins, polymethyl methacrylates, polyurethanes, polystyrenes, and glasses.

As shown in Fig. 3, the glass plate 34 has a coating layer 34A containing laminin in the areas exposed by the isolating pores 33A.

The material of the glass plate 34 is preferably a material which more easily allows formation of a coating layer than the materials of the first PDMS plate 32 and the second PDMS plate 33.

The microchannels 32B are channels formed by the resin plate 30, first PDMS plate 32, second PDMS plate 33, and glass plate 34 disposed one on another in layers in this order.

That is, each microchannel 32B has hollows (pores) in the main channel constituted by surfaces of the first PDMS plate 32 and the second PDMS plate 33, which hollows are constituted by the isolating pores 33A whose bottom surfaces are the glass plate 34.

The microchannels 32B have the coating layer 34A containing laminin. More specifically, for example, among the inner walls of each microchannel 32B, the glass plate 34 as the bottom surface exposed by the isolating pores 33A is mainly selectively coated with the coating layer 34A containing laminin.

By subjecting the surface of the glass plate 34 to a treatment to give hydrophilicity thereto, the selective coating of the surface with the coating layer containing laminin can be made easier. That is, the coating layer may be formed on the glass plate 34 constituting the bottom surface exposed by the isolating pores 33A, by a method including giving hydrophilicity to the surface of the glass plate 34 constituting the bottom surface and then injecting a solution for formation of the coating layer containing laminin into the microchannels 32B.

The coating layer 34A may contain Matrigel, fibronectin, poly-L-lysine, and/or the like in addition to laminin as long as the culture is not affected.

Examples of the method of forming the coating layer 34A include a method in which a cell culture member 12 having the constitution described above is constructed and microchannels 32B are formed, followed by injecting a solution for formation of a coating layer containing laminin from first holes 30A or second holes 30B into the microchannels to coat the glass plate 34. For enabling more selective formation of the coating layer 34A on the glass plate 34, the surface of the glass plate 34 may be treated with oxygen plasma or the like to obtain hydrophilicity.

More specifically, the solution for formation of a coating layer containing laminin is prepared as follows.

The solution is prepared by diluting a coating agent such as laminin with PBS or a culture medium.

From the viewpoint of singly seeding single cells, the cell culture member 12 described herein preferably has an isolating structure which isolates each single cell from other cells in the microchannels 32B as shown in Fig. 3.

The isolating structure is not limited to the structure shown in Fig. 3, and other examples of the isolating structure include a structure in which a pair of partition walls 33B are provided in each microchannel 32B (see Fig. 4), and a structure in which coating layers containing laminin 34A are partially provided on a flat surface (the coating layers partially provided in the microchannel 32B are hereinafter also referred to as "partial coating layers 34B"; see Fig. 5).

In the structure shown in Fig. 4, which has partition walls in each microchannel 32B, a plurality of partition walls 33B are provided. Therefore, single cells can be singly seeded such that each cell placed between the partition walls 33B is isolated from other cells, and the isolated single cells can be cultured. That is, the partition walls 33B function as an isolating structure that isolates each single cell from other cells.

In the structure shown in Fig. 5, in which coating layers containing laminin are partially provided, the partial coating layers 34B containing laminin are provided on a flat surface of the glass plate 34. By separately providing the plurality of partial coating layers 34B, each single cell on the partial coating layers 34B can be isolated from other cells.

From the viewpoint of singly seeding single cells, the cell culture member 12 preferably has an isolating structure. From the viewpoint of promoting culture of the singly seeded single cells, the isolating structure is preferably the structure having the isolating pores 33A in the microchannels 32B (structure shown in Fig. 3) or the structure having the partition walls 33B in the microchannels 32B (structure shown in Fig. 4).

It is thought that in the case in which the isolating structure is the structure having the isolating pores 33A in the microchannels 32B or the structure having the partition walls 33B in the microchannels 32B, the space in the vicinity of each singly seeded single cell is narrow, and new culture fluid is less likely to flow into the vicinity of the single cell, and that the phenomenon in which a substance (for example, a substance required for the growth) secreted from a cell acts on the cell itself from which the substance was secreted (autocrine) may therefore easily occur, whereby the cell can be easily grown.

The cell culture apparatus 10 disclosed herein is preferably placed in an incubator 102 (thermostat). For example, in the case of human-derived cells, the temperature of the culture fluid is preferably kept at from 36°C to 38°C, so that the cells can be cultured at a temperature near the body temperature of a human.

The incubator 102 is preferably an incubator that can maintain a temperature suitable for cell culturing. The cell culturing is preferably carried out using a CO₂ incubator 102 as the incubator 102, under a CO₂ atmosphere of from 4% to 6%.

For maintenance culturing, the cell culture apparatus may be placed under low-oxygen conditions. The term "low-oxygen conditions" herein means a state where the oxygen partial pressure is from 1% to 10%, preferably 5%.

The cell culture member can be used as a kit for testing the effect of a candidate agent by culturing singly dispersed pluripotent cells in a single-cell state and then further allowing a culture medium in which the candidate agent, which is the subject of screening, has been added to flow, followed by observing changes in the pluripotent cells.

Examples of the changes in the pluripotent cells herein include changes into specific cells such as endodermal cells, ectodermal cells, mesodermal cells, chorda-mesoderm, paraxial mesoderm, intermediate mesodermal cells, lateral plate mesodermal cells, nerve cells, glial cells, hematopoietic cells, hepatocytes, pancreatic beta cells, renal progenitor cells, endothelial cells, pericytes, epithelial cells, osteoblasts, myoblasts, or chondrocytes. The candidate agent can be selected as a differentiation inducer for differentiation into each type of cell.

In the case in which the culture member disclosed herein is used, culturing is carried out in the microchannel. Accordingly, the amount of the candidate agent can be reduced.

### EXAMPLES

### [Test Example 1]

### (Single-cell Dispersion Liquid 1)

### -Preparation of iPS Cells-

OCT3/4, SOX2, KLF4, and c-MYC were introduced to human embryonic lung fibroblasts (TIG1) provided by the JCRB Cell Bank using a retrovirus, to induce an hiPSC line. On a Matrigel-coated dish, the TIG1-iPSCs were cultured in an MEF (mouse embryonic fibroblast)-conditioned hES culture medium (DMEM/F12 supplemented with 20% knockout serum replacement (Invitrogen, Carlsbad, CA, USA), L-glutamine, non-essential amino acids, 2-mercaptoethanol, and 10 ng/ml bFGF (Peprotech, Rocky Hill, NJ, USA)).

### -Dispersion of Cells-

The TIG1-iPSCs subcultured in the culture medium containing Matrigel were detached using 0.25% trypsin (Gibco)/0.04% EDTA, and recovered. After washing the recovered cells twice, the cells were suspended in 500 µl of fresh culture fluid (MEF conditioned medium, mTeSR1 (modified Tenneille Serum Replacer 1)) such that the cell concentration became 5.0×10² cells/ml, to obtain a single-cell dispersion liquid 1.

### (Cell Culture Member)

### -Preparation of Members-

### • Resin Plate 30

A resin plate 30 was prepared with polycarbonate (PC). In the resin plate 30, first holes 30A and second holes 30B each having an inner diameter of 1 mm were provided.

### • First PDMS Plate 32

A thermosetting PDMS (SLIPOT 184, Toray-DawCorning, Japan) was fed into a mold for formation of 6 slits (0.5 mm width, 20 mm length, and 0.5 mm depth) to be used as microchannels, which mold was prepared by SU8 lithography, so as to obtain a thickness of 0.5 mm. The PDMS in the mold was then cured in a drier at 80°C for 4 hours. Subsequently, the cured PDMS was removed from the mold, to obtain a first PDMS plate 32 in which 6 channels were formed.

### • Second PDMS Plate 33

A second PDMS plate 33 was prepared in the same manner as the first PDMS plate 32 except that isolating pores 33A having an inner diameter of 1 mm and depth of 0.5 mm were formed instead of the 6 slits to be used as the microchannels in the first PDMS plate 32.

Five isolating pores 33A per each slit were provided in the second PDMS plate 33 such that the pores were positioned along the longitudinal direction of each groove to be used as a microchannel in the first PDMS plate 32. The pores were positioned such that the distance between the center of each pore and the center of a pore adjacent to this pore was 2.5 mm.

A total of 30 isolating pores 33A were provided in the second PDMS plate 33.

### • Glass Plate 34

On a glass plate, an SU8 layer was formed by application of SU8 (SU-8 2010, manufactured by Microchem), to provide a glass plate 34. The SU8 layer was prepared by applying SU8 to the glass plate and then curing the SU8 by UV irradiation. A cross mark was given by SU8 lithography for adjustment of the XY axis during attachment to the first and second PDMS layers. Subsequently, the surface of the SU8 layer was treated with oxygen plasma to add hydrophilicity thereto.

### -Assembly of Cell Culture Member-

The cell culture member 12 shown in Fig. 2 was obtained by the following method.

The resin plate 30; the first PDMS plate 32 having slit-shaped grooves forming microchannels; the second PDMS plate 33 forming an isolating structure (the structure in which isolating pores 33A are present in the microchannels of (1)); and the glass plate 34 having the coating layer; were disposed one on another in layers from the top to form a laminated body. A lower clamp was placed under the glass plate of the laminated body, and an upper clamp was placed over the resin plate, such that the resin plate 30, the first PDMS plate 32, the second PDMS plate 33, and the glass plate 34 were sandwiched between the upper clamp and the lower clamp.

In this state, bolts were inserted through bolt holes formed in the upper clamp and bolt holes formed in the lower clamp to tightly bind the clamps to each other, to thereby prepare a cell culture member 12 on which a coating layer has not been formed yet.

### -Formation of Coating Layer-

First, a solution for formation of a coating layer containing human laminin 521 was prepared as follows.

Human laminin 521 was diluted with PBS to prepare a solution at a final concentration of 20 µg/mL.

The solution for formation of a coating layer was injected from the first holes 30A (inlets) of the cell culture member 12 obtained as described above, to form a coating layer containing human laminin 521 such that the layer coats the glass plate 34 exposed by the isolating pores 33A in the microchannels 32B.

As a result of observation using a phase contrast microscope, the coating layer was formed on the areas exposed by the isolating pores 33A in the microchannels 32B, that is, on the glass plate 34.

### (Culture of Single Cells)

### -Seeding Process-

From the first holes 30A (inlets) of the cell culture member after the formation of the coating layer containing human laminin 521, the single-cell dispersion liquid 1 was injected using a syringe, to singly seed the single cells. The injection of the single-cell dispersion liquid 1 was carried out such that the grooves in the first PDMS plate 32 and the pores provided in the second PDMS plate 33 were filled with the dispersion liquid.

The number of single cells seeded in the microchannels was 165.

After the injection, it was confirmed using a phase-contrast microscope that a single cell was placed in each of the isolating pores 33A provided on the second PDMS plate 33.

Thereafter, the cell culture member in which the single cells were singly seeded was left to stand for 24 hours in the CO₂ incubator 102 at a constant temperature of 37°C.

### -Circulation Process-

The cell culture member which had been left to stand was placed in the cell culture apparatus 10 shown in Fig. 1.

As the tubes for connection, polytetrafluoroethylene (PTFE) tubes of TUF-100 series AWG-30 (manufactured by Chukoh Chemical Industries, Japan) were used.

A conditioned culture fluid prepared by culturing TIG1-iPSCs in the same kind of culture fluid as used for the single-cell dispersion liquid 1 was placed to the reservoir 14.

Using the cell culture apparatus, the conditioned culture fluid was circulated at a flow rate of 5000 nl/minute. The cell culture apparatus was placed in the CO₂ incubator 102 at a constant temperature of 37°C.

### (Evaluation)

### -Confirmation of Culture-

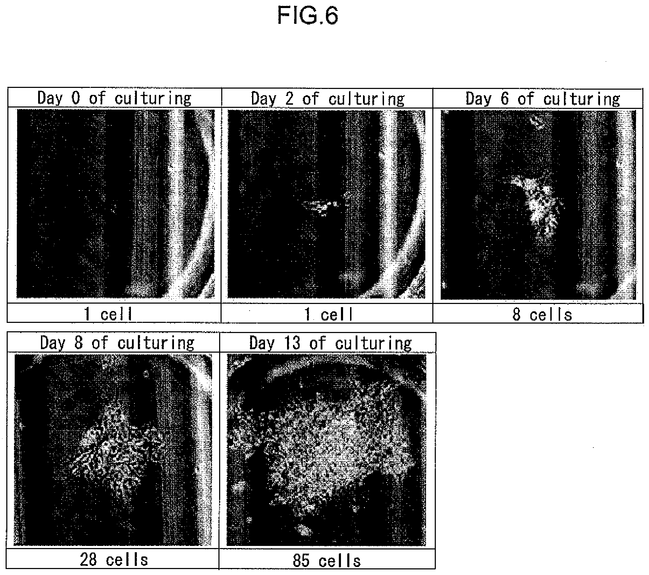

Using a phase-contrast microscope, confirmation of the culture was carried out by observation of the single cells that were singly seeded with the cell culture apparatus. The observation was carried out a total of 5 times: immediately before the start of the circulation (Day 0), on the second day of the culturing (Day 2 of the culturing), on the 6th day of the culturing (Day 6 of the culture), on the 8th day of the culturing (Day 8 of the culture), and on the 13th day of the culturing (Day 13 of the culture). The cell number was 1 on Day 0, 1 on Day 2, 8 on Day 6, 28 on Day 8, and 85 on Day 13. The observation results are shown in Fig. 6.

As a result of the observation, growth of a single cell to not less than 50 cells was observed in 2 cases ("Number of single cells showing growth to not less than 50 cells (B)" in Table 1). From "Number of single cells seeded (A)" and "Number of single cells showing growth to not less than 50 cells (B)" in Table 1, the mean growth rate (B/A) was determined. The results are shown in Table 1.

### -Confirmation of Pluripotency-

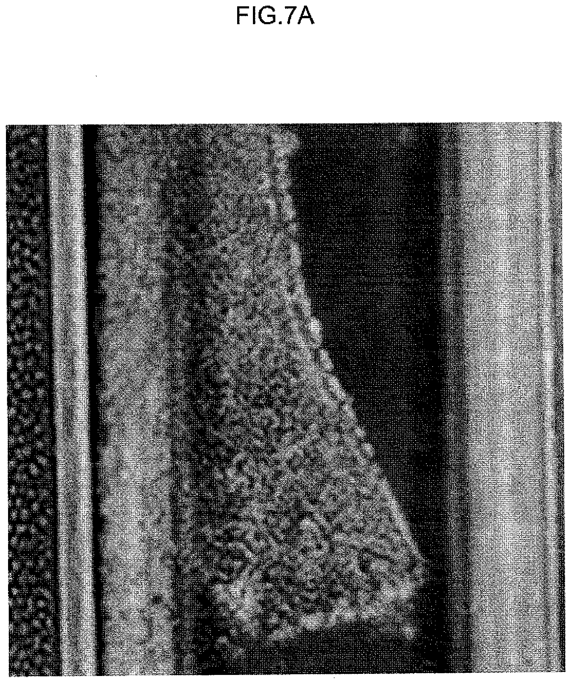

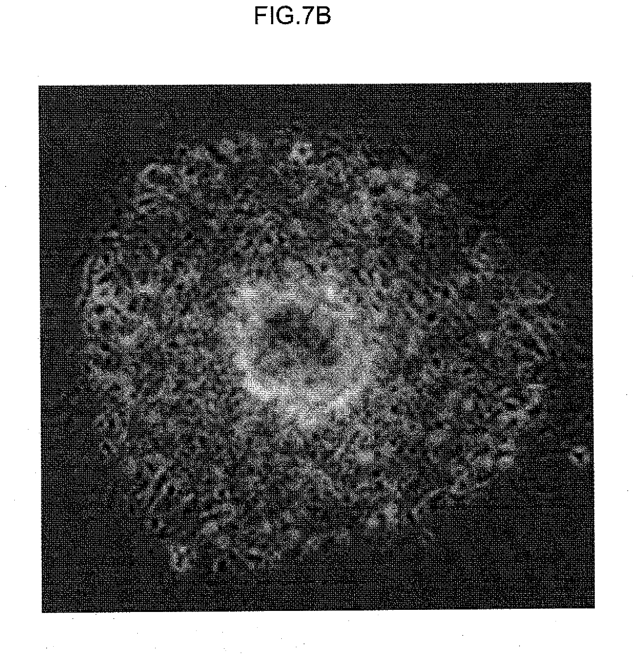

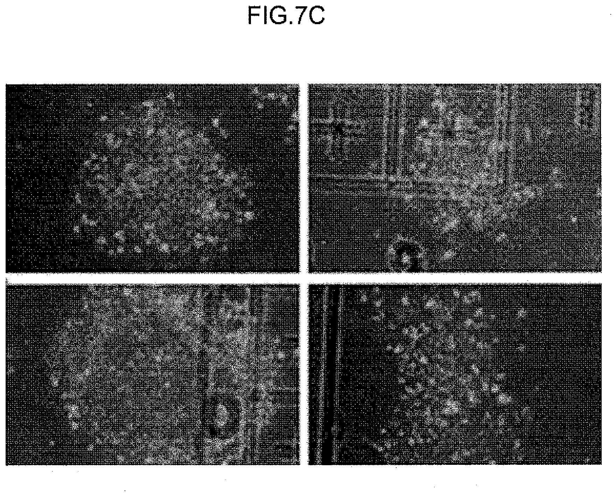

TIG1-iPSCs were taken from a colony of TIG1-iPSCs on Day 14 of the culture in the microchannels (see Fig. 7A), and subjected to normal culture on a Matrigel-coated plate (see Fig. 7B). The TIG1-iPSCs after performing the first subculture (see Fig. 7C) were recovered, and whether or not the cells maintained pluripotency was investigated.

The pluripotency was investigated by immunostaining. The methods and the results obtained thereby are described below.

### • Immunostaining

The recovered cells (TIG1-iPSCs) were fixed using 4% PFA (paraformaldehyde)/PBS (phosphate buffered saline) at room temperature (25°C) for 10 minutes, and washed with PBST (0.1% Triton X-100 in PBS), followed by performing pretreatment in a blocking solution (3% BSA and 2% skim milk (DIFCO, USA) in PBST) at 4°C overnight. Subsequently, immunoreaction was performed with a primary antibody: anti-OCT4 (1:50, Santa Cruz Biotechnology, USA), anti-SOX2 (1:500, Abcam, Cambridge, UK), or anti-NANOG (1:200, Abcam, Cambridge, UK), and staining was then performed with a fluorescent secondary antibody (1:500, Invitrogen).

Nuclei were counterstained with DAPI (4,6-diamidino-2-phenylindole), and photofading was prevented using SlowFade light antifade kit (Invitrogen), followed by obtaining fluorescent images using an 1X70 inverted microscope.

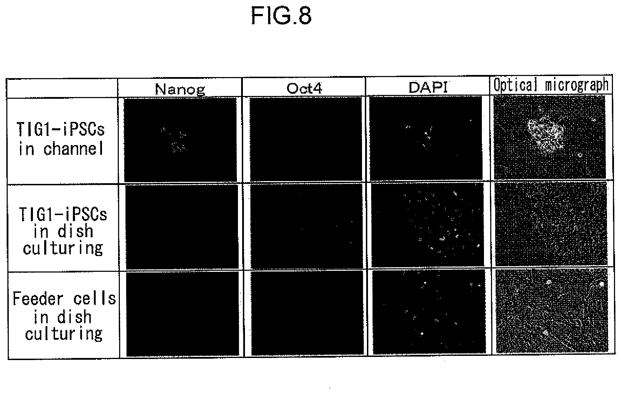

As a result of the immunostaining by the method described above, expression of pluripotency marker proteins OCT4 and NANOG was shown (see Fig. 8). As a result of the DAPI staining, nuclei were found.

From the above results, the culture method of the present Example was found to be capable of culturing the singly seeded single cells (TIG1-iPSCs) while maintaining the pluripotency of the cells.

As a Reference Example, TIG1-iPS and feeder cells (MEF, ReproCELL Incorporated) cultured in petri dishes instead of the microchannels were subjected to immunostaining in the same manner as described above (see Fig. 8).

As a result, similarly to the TIG1-iPS cells on Day 14 of the culturing in the microchannels in Test Example 1, the TIG1-iPS cells cultured in the petri dish showed expression of the pluripotency marker proteins OCT4 and NANOG. As a result of the DAPI staining, nuclei could be found.

With respect to the feeder cells, nuclei were found as a result of the DAPI staining, but the cells did not show expression of either the pluripotency marker protein OCT4 or the pluripotency marker protein NANOG.

### [Test Example 2]

TIG1-iPSCs were obtained in the same manner as in Test Example 1 except that the prepared TIG1-iPSCs were cultured once in a culture medium containing human laminin 521 using a human laminin 521 coating, instead of the culture medium containing Matrigel using the Matrigel coating.

The TIG1-iPSCs obtained by this culturing were detached by the same treatment as in the preparation of the single-cell dispersion liquid 1, to obtain a single-cell dispersion liquid 2. The cell concentration in the single-cell dispersion liquid 2 was 5×10² cells/ml.

The single-cell dispersion liquid 2 was seeded by injection into the same cell culture member as in Test Example 1, and culturing was carried out in the same manner as in Test Example 1.

After injection of the single-cell dispersion liquid 2, it was confirmed using a phase-contrast microscope that a single cell was placed in each of the pores provided on the second PDMS plate 33.

The number of single cells seeded in the microchannels in Test Example 2 was 262.

### (Evaluation)

The cultured single cells were observed in the same manner as in Test Example 1 using a phase-contrast microscope. As a result, growth of a single cell to not less than 50 cells was observed in 10 cases. From "Number of single cells seeded (A)" and "Number of single cells showing growth to not less than 50 cells (B)" in Table 1, the mean growth rate (B/A) was determined. The results are shown in Table 1.

### [Comparative Test Example 1]

A cell culture member was prepared in the same manner as in the preparation of the cell culture member used in Test Example 1 except that a solution for formation of a coating layer containing Matrigel, instead of human laminin 521, was used.

The solution for formation of a Matrigel-containing coating layer was prepared as follows.

Matrigel was 50-fold diluted with the culture fluid to prepare a Matrigel coating solution.

Thereafter, the single-cell dispersion liquid 1 was seeded by injection into the cell culture member in the same manner as in Test Example 1, and culturing was carried out in the same manner as in Test Example 1.

After the injection of the single-cell dispersion liquid 1, it was confirmed using a phase-contrast microscope that a single cell was placed in each of the pores provided on the second PDMS plate 33.

The number of single cells seeded in the microchannels in Comparative Test Example 1 was 783.

### [Evaluation]

The cultured single cells were observed in the same manner as in Test Example 1 using a phase-contrast microscope. As a result, growth of a single cell to not less than 50 cells was not observed. From "Number of single cells seeded (A)" and "Number of single cells showing growth to not less than 50 cells (B)" in Table 1, the mean growth rate (B/A) was determined. The results are shown in Table 1.

**Table 1**

| | Coating for subculture medium | Coating layer in microchannels | Number of single cells seeded (A) | Number of single cells showing growth to not less than 50 cells (B) | Mean growth rate (B/A) (%) |
|---|---|---|---|---|---|
| Test Example 1 | Matrigel | Human laminin 521 | 165 | 2 | 1.2 |
| Test Example 2 | Human laminin 521 | Human laminin 521 | 262 | 10 | 3.8 |
| Comparative Test Example 1 | Matrigel | Matrigel | 783 | 0 | 0 |

Based on the results, it is clear that the cell culture method, cell culture member, and cell culture apparatus of the present Examples enable culturing of singly seeded single cells and producing clone cells of these cells while maintaining their pluripotency.

It also became clear that inclusion of human laminin 521 is desirable not only in the coating layer of the microchannels, but also in the medium to be used for subculture of cells in preparation of the single-cell dispersion liquid.

## Claims

1. A cell culture method comprising:
a seeding process of singly seeding single pluripotent cells on a coating layer of a microchannel, the coating layer being present on an inner wall of the microchannel, and the coating layer containing laminin; and
a circulation process of circulating a culture fluid in the microchannel where the single pluripotent cells have been singly seeded in the seeding process, wherein said pluripotency is maintained during said method.

2. The cell culture method according to claim 1, wherein the single pluripotent cells seeded in the seeding process are cells that have been subcultured in a medium containing laminin.

3. The cell culture method according to claim 2, wherein the number of subculturing passages is not less than 2.

4. The cell culture method according to any one of claims 1 to 3, wherein the culture fluid is a conditioned culture fluid.

5. The cell culture method according to any one of claims 1 to 4, wherein, in the circulation process, the circulation of the culture fluid is started after maintaining the microchannel, in which the single pluripotent cells have been singly seeded in the seeding process, in a state where the inside of the microchannel is filled with the culture fluid, for a predetermined time period.

6. The cell culture method according to any one of claims 1 to 5, wherein the laminin is human laminin.

7. The cell culture method according to any one of claims 1 to 6, wherein the microchannel has, in the inside thereof, an isolating structure which isolates each single pluripotent cell from other cells.

8. The cell culture method according to any one of claims 1 to 7 wherein said cells are induced pluripotent stem cells.

## Patentansprüche

1. Zellkulturverfahren, umfassend:
einen Impfprozess des einzelnen Beimpfens einzelner pluripotenter Zellen auf einer Überzugsschicht von einem Mikrokanal, wobei die Überzugsschicht auf einer Innenwand des Mikrokanals vorhanden ist und die Überzugsschicht Laminin enthält; und
einen Zirkulationsprozess des Zirkulierens eines Kulturfluids in dem Mikrokanal, wobei die einzelnen pluripotenten Zellen in dem Impfprozess einzeln beimpft worden sind, wobei die Pluripotenz während es Verfahrens aufrechterhalten wird.

2. Zellkulturverfahren nach Anspruch 1, wobei die einzelnen pluripotenten Zellen, die in dem Impfprozess beimpft werden, Zellen sind, die in einem Laminin enthaltendem Medium subkultiviert worden sind.

3. Zellkulturverfahren nach Anspruch 2, wobei die Zahl der subkultivierenden Passagen nicht weniger als 2 beträgt.

4. Zellkulturverfahren nach einem der Ansprüche 1 bis 3, wobei das Kulturfluid ein konditioniertes Kulturfluid ist.

5. Zellkulturverfahren nach einem der Ansprüche 1 bis 4, wobei die Zirkulation des Kulturfluids in dem Zirkulationsprozess gestartet wird, nachdem der Mikrokanal, in welchem die einzelnen pluripotenten Zellen in dem Impfprozess einzeln beimpft worden sind, für eine vorbestimmte Zeit in einem Zustand gehalten wird, in dem die Innenseite des Mikrokanals mit dem Kulturfluid gefüllt ist.

6. Zellkulturverfahren nach einem der Ansprüche 1 bis 5, wobei das Laminin humanes Laminin ist.

7. Zellkulturverfahren nach einem der Ansprüche 1 bis 6, wobei der Mikrokanal an der Innenseite davon eine isolierende Struktur aufweist, die jede einzelne pluripotente Zelle gegenüber anderen Zellen isoliert.

8. Zellkulturverfahren nach einem der Ansprüche 1 bis 7, wobei die Zellen induzierte pluripotente Stammzellen sind.

## Revendications

1. Procédé de culture cellulaire comprenant :
une opération d'ensemencement consistant à semer individuellement des cellules pluripotentes individuelles sur une couche d'enrobage d'un microcanal, la couche d'enrobage se présentant sur une paroi interne du microcanal, et la couche d'enrobage contenant de la laminine ; et
une opération de circulation consistant à faire circuler un liquide de culture dans le microcanal où les cellules pluripotentes individuelles ont été semées individuellement lors de l'opération d'ensemencement, ladite pluripotence étant maintenue durant ledit procédé.

2. Procédé de culture cellulaire selon la revendication 1, dans lequel les cellules pluripotentes individuelles semées lors de l'opération d'ensemencement sont des cellules qui ont été repiquées dans un milieu contenant de la laminine.

3. Procédé de culture cellulaire selon la revendication 2, dans lequel le nombre de passages de repiquage est supérieur ou égal à 2.

4. Procédé de culture cellulaire selon l'une quelconque des revendications 1 à 3, dans lequel le liquide de culture est un liquide de culture conditionné.

5. Procédé de culture cellulaire selon l'une quelconque des revendications 1 à 4, dans lequel, lors de l'opération de circulation, la circulation du liquide de culture est lancée après maintien du microcanal, dans lequel les cellules pluripotentes individuelles ont semées individuellement lors de l'opération d'ensemencement, dans un état où la partie intérieure du microcanal est remplie du liquide de culture, pendant une durée prédéterminée.

6. Procédé de culture cellulaire selon l'une quelconque des revendications 1 à 5, dans lequel la laminine est une laminine humaine.

7. Procédé de culture cellulaire selon l'une quelconque des revendications 1 à 6, dans lequel le microcanal comporte, dans sa partie intérieure, une structure isolante qui isole chaque cellule pluripotente individuelle des autres cellules.

8. Procédé de culture cellulaire selon l'une quelconque des revendications 1 à 7 dans lequel lesdites cellules sont des cellules souches pluripotentes induites.
